# DEMANDE DE BREVET EUROPEEN

(11) **EP 1 064 929 A1**
(43) Date de publication de la demande: **03.01.2001**
(21) Numéro de dépôt: 00401513.7
(22) Date de dépôt: 29.05.2000
(51) Int. Cl.: A61K 7/48

(54) **Utilisation d'huile fluorée volatile pour estomper les imperfections et matifier la peau**

(30) Priorité: 01.07.1999 FR 9908490
(71) Demandeur: L'OREAL, 75008 Paris (FR)
(72) Inventeur: Lorant, Raluca, 94320 Thiais (FR)
(74) Mandataire: Dodin, Catherine

(57) **Abrégé**

La présente demande concerne l'utilisation d'au moins une huile fluorée volatile dans une composition cosmétique ou pour la préparation d'une composition dermatologique, pour estomper et/ou camoufler les imperfections et/ou les défauts du relief de la peau tels que les rides, les ridules, les pores et/ou les microreliefs; et/ou pour matifier la peau; et/ou pour unifier et/ou éclaircir le teint.

L'invention concerne également un procédé de traitement cosmétique de la peau destiné à lui conférer un aspect plus mat et/ou à estomper et/ou camoufler les imperfections et/ou les défauts du relief de la peau tels que les rides, les ridules, les pores et/ou les microreliefs; et/ou à unifier et/ou éclaircir le teint, à l'aide d'une composition cosmétique comprenant au moins une huile fluorée volatile.

## Description

La présente invention a trait à l'utilisation, notamment dans une composition de soin ou de maquillage de la peau, d'une huile fluorée volatile, afin d'améliorer l'apparence de la peau, notamment de la rendre plus mate.

Par définition, un produit matifiant est un produit qui empêche la peau de briller et qui unifie le teint. Les compositions de soin ou de maquillage de la peau ayant des propriétés matifiantes sont généralement utilisées pour résoudre les problèmes de brillance occasionnés par un excès de sébum et pour améliorer la tenue du maquillage à long terme qui a tendance à se dégrader visuellement au cours de la journée.
Elles donnent un aspect mat à la peau résultant d'un pouvoir diffusant de la lumière à la surface de la peau. Elles peuvent aussi être utilisées pour estomper les défauts ou imperfections de la peau tels que les microreliefs, les rides, les ridules, les pores ou les variations de couleur.
Par ailleurs, l'effet de matité est particulièrement recherché pour les utilisateurs à peaux mixtes ou grasses, ainsi que sous les climats chauds et humides.

Les compositions classiques dites matifiantes contiennent généralement des poudres adsorbant le sébum et l'huile excédentaire de la composition non adsorbée par la peau. Parmi les poudres matifiantes d'origine naturelle ou synthétique, on peut citer notamment les charges telles que le talc, l'amidon, le mica, la silice, les poudres de Nylon, les poudres de polyéthylène, la poly-beta-alanine, les poudres de poly-(méth)acrylate de méthyle. Ce type de charges présente toutefois l'inconvénient de donner à la peau un aspect poudreux, peu naturel, qui peut même accentuer ses défauts. De plus, ces compositions sont généralement desséchantes à long terme et s'étalent difficilement. Leur effet matifiant est peu durable dans le temps.
Le document EP-A-502769 décrit des compositions matifiantes apportant une couche translucide et un aspect naturel à la peau maquillée. Il s'agit de dispersions de particules sphériques dans un liant gras dans un rapport en poids charges/liant très spécifique. Pour avoir un effet matifiant, il faut une forte proportion de poudres et, de ce fait, ces compositions peuvent être desséchantes. En outre, elles ont tendance à pelucher lors de l'étalement et à donner un effet blanchissant à la peau en raison de la forte concentration en poudres.
Plus récemment, on a proposé d'utiliser des polymères matifiants tels que des polymères siliconés réticulés connus sous les références commerciales KSG de la société Shin Etsu, Tréfils de la société Dow Corning ou Gransils de la société Grand Industrie. L'inconvénient de ces produits commerciaux est de contenir des huiles de silicones linéaires ou cycliques du type polydiméthylsiloxanes (PDMS) non réticulés et d'apporter un effet huileux, gras, sans effet frais, ce qui ne permet pas ou difficilement leur utilisation en milieu chaud et humide et/ou par les utilisateurs à peaux grasses. De plus certains de ces produits commerciaux sont difficilement dispersibles dans un milieu aqueux.

Il subsiste donc le besoin d'une composition matifiante, dont les propriétés persistent sur la peau au cours du temps, et qui apportent en même temps des propriétés de fraîcheur.
La Demanderesse a découvert de manière surprenante que l'incorporation d'une huile fluorée particulière dans une composition notamment cosmétique, permettait de résoudre ces problèmes et donc de camoufler les imperfections du relief de la peau et/ou de la rendre plus mate ou moins brillante, bien que l'on utilise un corps gras.

Un objet de la présente invention est donc l'utilisation d'au moins une huile fluorée volatile dans une composition cosmétique ou pour la préparation d'une composition dermatologique, pour estomper et/ou camoufler les imperfections et/ou les défauts du relief de la peau tels que les rides, les ridules, les pores et/ou les microreliefs; et/ou pour matifier la peau; et/ou pour unifier et/ou éclaircir le teint.

Un autre objet de l'invention est un procédé de traitement cosmétique de la peau du visage, du corps y compris le cuir chevelu, des lèvres, destiné à lui conférer un aspect plus mat et/ou à estomper et/ou camoufler les imperfections et/ou les défauts du relief de la peau tels que les rides, les ridules, les pores et/ou les microreliefs; et/ou à unifier et/ou éclaircir le teint, caractérisé par le fait qu'on applique sur la peau une quantité efficace d'une composition cosmétique comprenant au moins une huile fluorée volatile.

Les compositions obtenues selon l'invention sont fraîches à l'application; elles s'étalent facilement et ne peluchent pas sur la peau ou les lèvres. Elles sont parfaitement adaptées aux peaux grasses, du fait de leur haut pouvoir matifiant et de leur texture légère, évanescente et fraîche.
Par ailleurs, on a constaté que les compositions selon l'invention avaient un effet assainissant pour les peaux grasses et brillantes. Après application, la peau paraît plus claire et plus nette, plus lumineuse; le teint est éclairci. Ceci peut être dû aux propriétés solvantes des huiles fluorées volatiles, qui débarrassent la peau grasse de l'excès de sébum susceptible de provoquer des comédons ou des boutons qui ternissent la peau.

L'huile fluorée volatile susceptible d'être employée dans le cadre de la présente invention présente de préférence une densité supérieure à environ 1, par exemple supérieure à environ 1,1, notamment supérieure à environ 1, 2. Elle peut avoir une pression de vapeur saturante, à 25°C, au moins égale à 50 Pa, par exemple supérieure à 2000 Pa, de préférence supérieure à 4000 Pa.

On peut notamment citer, seul ou en mélange, :
i) les perfluorocycloalkyles répondant à la formule (I) suivante : 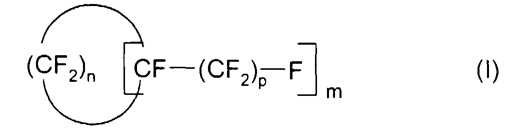 dans laquelle :
   - n est 4 ou 5,
   - m est 1 ou 2, et
   - p est 1, 2 ou 3,
   sous réserve que lorsque m = 2, les groupements ne sont pas nécessairement en alpha, l'un par rapport à l'autre;
ii) les perfluoroalcanes répondant à la formule (II) suivante :

   CF₃-(CF₂)ₘ-CF₂X (II)

   dans laquelle m est 2 à 8 et X représente Br ou F;
iii) les fluoroalkyles ou hétérofluoroalkyles répondant à la formule (III) suivante :

   CH₃-(CH₂)ₙ-[Z]ₜ-(CF₂)ₘ-CF₃ (III)

   dans laquelle :
   - t est 0 ou 1,
   - n est 0, 1, 2 ou 3,
   - m est 2, 3, 4 ou 5, et
   - Z est O, S ou NR avec R représentant H, -(CH₂)ₘ-CH₃ ou -(CF₂)ₘ-CF₃.
iv) les dérivés de perfluoromorpholine répondant à la formule (IV) : 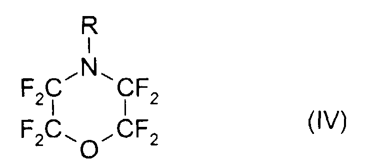 dans laquelle R est un radical perfluoroalkyle en C1-C4.

Parmi les perfluorocycloalkyles de formule (I), on peut notamment citer le perfluorométhylcyclopentane et le perfluoro-1,3-diméthylcyclohexane vendus sous les dénominations de "FLUTEC PC1®" et "FLUTEC PC3®" par la Société BNFL FLUOROCHEMICALS Ltd.
Parmi les perfluoroalcanes de formule (II), on peut notamment citer le dodécafluoropentane et le tétradécafluorohexane vendus sous les dénominations de "PF5050®" et "PF5060®" par la Société 3M, ou encore le bromoperfluorooctyle vendu sous la dénomination "FORALKYL" par la société ATOCHEM.
Parmi les composés fluorés de formule (III) on peut notamment citer le nonafluorométhoxybutane vendu sous la dénomination de "MSX 4518®" par la société 3M et le nonafluoroéthoxyisobutane.
Parmi les composés de formule (IV), on peut notamment citer la 4-trifluorométhyl perfluoromorpholine vendue sous la dénomination "PF5052®" par la société 3M.

Ces huiles fluorées volatiles peuvent être présentes dans les compositions cosmétiques ou dermatologiques à raison de 0,5 à 100% en poids, notamment 3 à 40% en poids et de préférence 5 à 20% en poids.

Lesdites compositions peuvent se présenter sous toutes les formes galéniques envisageables, telles que émulsion huile-dans-eau, eau-dans-huile ou multiple, plus ou moins fluide; produit anhydre tel que solution, dispersion ou gel anhydre lipophile, monophase, biphase ou multiphase. On entend ici par "émulsion" aussi bien des dispersions sans émulsionnants que des dispersions comportant des émulsionnants ou encore des dispersions stabilisées par des particules solides ou par des sphérules lipidiques de type ionique ou non ionique. Pour une utilisation pour les peaux grasses, on préfère avoir une émulsion H/E.

Ainsi, en fonction de la forme galénique et de l'utilisation envisagées, l'homme du métier choisira les autres ingrédients de la composition sur la base de ses connaissances.
Notamment, la composition peut comprendre d'autres huiles, fluorées ou non, volatiles ou non, ainsi que tout corps gras usuellement utilisé dans le domaine d'application envisagé. On peut notamment citer les corps gras siliconés tels que les huiles, les corps gras pâteux, les gommes et les cires de silicone, ainsi que les corps gras non siliconés tels que les huiles, les pâteux et les cires végétales, minérales, animales et/ou synthétiques.
On peut notamment citer :
- les huiles hydrocarbonées d'origine animale telles que le perhydrosqualène ;
- les huiles hydrocarbonées d'origine végétale telles que les triglycérides liquides d'acides gras, par exemple les huiles de tournesol, de maïs, de soja, de courge, de pépins de raisin, de jojoba, de sésame, de noisette, d'abricot, de macadamia, de ricin, d'avocat, d'olive, de germes de blé, d'amande douce, de calophyllum, de palme; les triglycérides des acides caprylique/caprique comme ceux vendus par la société Stearineries Dubois ou ceux vendus sous les dénominations Miglyol 810, 812 et 818 par la société Dynamit Nobel ;
- les huiles de formule R¹COOR² dans laquelle R¹ représente le reste d'un acide gras supérieur comportant de 7 à 19 atomes de carbone et R² représente une chaîne hydrocarbonée ramifiée contenant de 3 à 20 atomes de carbone comme par exemple l'huile de Purcellin ;
- les hydrocarbures linéaires ou ramifiés, d'origine minérale ou synthétique tels que les huiles de paraffine volatiles ou non et leurs dérivés, la vaseline, les polydécènes, le polyisobutène hydrogéné tel que le parléam ;
- les esters et les éthers de synthèse comme le myristate d'isopropyle, des octanoates, décanoates ou ricinoléates d'alcools ou de polyalcools ;
- des alcools gras comme l'octyldodécanol ou l'alcool oléique ;
- les polyalkyl(C₁-C₂₀) siloxanes et notamment ceux à groupements terminaux triméthylsilyle, de préférence ceux dont la viscosité est inférieure à 0,06 m²/s parmi lesquels on peut citer les polydiméthylsiloxanes linéaires et les alkylméthylpolysiloxanes tels que la cétyldiméthicone (nom CTFA),
- les huiles siliconées volatiles, telles que les silicones volatiles cycliques ayant de 3 à 8 atomes de silicium et de préférence de 4 à 5, notamment la cyclotétradiméthylsiloxane, de la cyclopentadiméthylsiloxane ou de la cyclohexadiméthylsiloxane; les cyclocopolymères du type diméthylsiloxane/méthylalkylsiloxane tels que le cyclocopolymère diméthylsiloxane/méthyloctylsiloxane (SILICONE FZ 3109 de UNION CARBIDE); les silicones volatiles linéaires ayant de 2 à 9 atomes de silicium tels que l'hexaméthyldisiloxane, l'hexylheptaméthyltrisiloxane et l'octylheptaméthyltrisiloxane; - les huiles de silicone phénylées, notamment celles de formule : 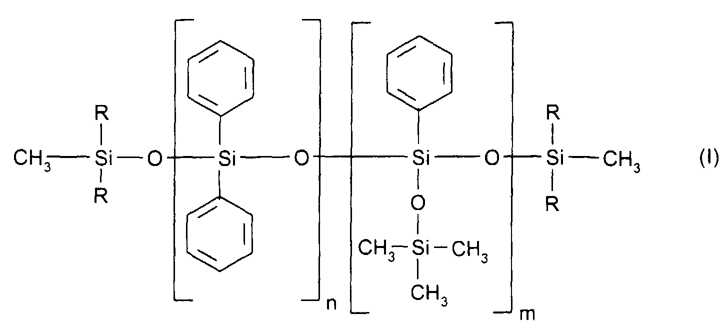 dans laquelle R est un radical alkyle en C1-C30, un radical aryle ou un radical aralkyle; et m et n sont, indépendamment l'un de l'autre, des entiers compris entre 0 et 100, sous réserve que la somme m+n est comprise entre 1 et 100;
- les gommes de silicones;
- les huiles volatiles hydrocarbonées, telles que les isoparaffines et notamment l'isododécane et l'isohexadécane.

Par ailleurs, la composition selon l'invention peut contenir des cires hydrocarbonées, fluorées ou siliconées ou leurs mélanges, qui peuvent être solides ou semi-solides (sous forme d'une pâte) à température ambiante. Ces cires peuvent être d'origine végétale, minérale, animale et/ou synthétique. En particulier, ces cires présentent une température de fusion supérieure à 25°C et mieux supérieure à 45°C. La présence de cires permet d'assurer une bonne résistance mécanique, notamment lorsque la composition se présente sous la forme d'un stick.
On peut notamment citer les cires de silicone, les huiles hydrogénées concrètes à 25°C; les cires de lanoline; les esters gras concrets à 25°C; la cire d'abeilles; les cires végétales telles que la cire de Carnauba, de Candellila, d'Ouricury, du Japon ou les cires de fibres de lièges ou de canne à sucre; les cires minérales, par exemple de paraffine, de lignite ou les cires microcristallines ou les ozokérites; les cires synthétiques, parmi lesquelles les cires de polyéthylène et les cires obtenues par synthèse de Fischer-Tropsch.

Ces corps gras peuvent en particulier être choisis de manière variée par l'homme du métier afin de préparer une composition ayant les propriétés souhaitées, par exemple en consistance ou en texture.

Lorsque la composition se présente sous forme d'émulsion, elle peut comprendre au moins un émulsionnant classique, choisi parmi les émulsionnants amphotères, anioniques, cationiques ou non ioniques, utilisés seuls ou en mélange.

La composition peut également comprendre une phase particulaire, qui peut comprendre des pigments et/ou des nacres et/ou des charges habituellement utilisés dans les compositions cosmétiques. Par pigments, il faut comprendre des particules blanches ou colorées, minérales ou organiques, insolubles dans le milieu de la composition, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent à modifier la texture de la composition ainsi que l'effet de matité/brillance. Comme pigments minéraux, on peut citer les oxydes de titane, de zirconium ou de cérium ainsi que les oxydes de zinc, de fer ou de chrome et le bleu ferrique. Parmi les pigments organiques, on peut citer le noir de carbone, et les laques de baryum, strontium, calcium, aluminium. Parmi les nacres, on peut citer le mica recouvert d'oxyde de titane, d'oxyde de fer, de pigment naturel ou d'oxychlorure de bismuth tel que le mica titane coloré. Parmi les charges, on peut notamment citer le talc, le mica, la silice, le kaolin, les poudres de Nylon (Orgasol notamment) et de polyéthylène, le Téflon, l'amidon, le nitrure de bore, des microsphères de copolymères telles que l'Expancel (Nobel Industrie), le Polytrap (Dow Corning) et les microbilles de résine de silicone (Tospearl de Toshiba, par exemple).

La composition de l'invention peut comprendre, en outre, tout additif usuellement utilisé dans le domaine concerné, tel que des colorants, des antioxydants, des huiles essentielles, des conservateurs, des actifs cosmétiques ou dermatologiques comme des hydratants (glycérine), des vitamines, des acides gras essentiels, des filtres solaires, des polymères liposolubles notamment hydrocarbonés tels que les polyalkylènes, des gélifiants, des parfums.
Bien entendu l'homme du métier veillera à choisir ce ou ces éventuels additifs complémentaires et/ou leur quantité de manière telle que les propriétés avantageuses de la composition selon l'invention ne soient pas, ou substantiellement pas, altérées par l'adjonction envisagée. En particulier, ces additifs ne devront pas nuire à l'homogénéité, la stabilité, le confort, la matité et la fraîcheur de la composition.

La composition de l'invention est destinée à une application topique et comprend de manière appropriée un milieu physiologiquement acceptable. Elle trouve notamment une application dans le traitement cosmétique de la peau, et notamment en vue d'estomper les imperfections du relief de la peau, en particulier de camoufler les microreliefs, les rides et les ridules, les pores. Du fait de ses propriétés matifiantes, elle est également particulièrement appropriée pour le traitement des peaux grasses.
Bien entendu la composition doit être cosmétiquement ou dermatologiquement acceptable, à savoir non toxique et susceptible d'être appliquée sur la peau (y compris l'intérieur des paupières) ou les lèvres d'être humains.

La composition selon l'invention peut se présenter sous la forme d'un produit coloré ou non coloré, contenant éventuellement des actifs cosmétiques ou dermatologiques.
Elle peut notamment se présenter sous forme d'un produit de maquillage de la peau du visage, du corps ou des lèvres, tel qu'un fond de teint, un fard à joues ou à paupières, un stick anti-cernes, un eye-liner, un mascara, un rouge à lèvres.
Elle peut également se présenter sous forme d'un produit de soin dermatologique ou cosmétique de la peau du visage, du corps y compris le cuir chevelu, des lèvres, tel qu'une base de soin pour les lèvres (baumes à lèvres, protégeant les lèvres du froid et/ou du soleil et/ou du vent), une base fixante à appliquer sur un rouge à lèvres classique, notamment pour en limiter le transfert; une composition de protection solaire ou de bronzage artificiel; un produit déodorant; une composition de soin de jour pour le visage; une composition matifiante pour le visage.

L'invention est illustrée plus en détail dans les exemples suivants.

### Exemple 1 : Crème hydratante matifiante

| *phase A* | |
|---|---|
| dimethicone copolyol acétylméthyltaurate | 5,4% |
| glycérol | 3% |
| polymère d'AMPS | 2% |
| conservateurs | qs |
| eau | qsp 100% |

| *phase B* | |
|---|---|
| nonafluoromethoxybutane | 10% |
| cyclohexasiloxane | 10% |

On disperse à froid sous agitation la phase B dans la phase A et l'on obtient une crème matifiante d'application aisée et agréable.

### Exemple 2 : Lotion biphase assainissante rafraîchissante

| *phase supérieure* | |
|---|---|
| Isohexadecane | 50% |

| *phase inférieure* | |
|---|---|
| trifluoromethyl perfluoromorpholine | 25% |
| dodecafluoropentane | 25% |

On mélange la phase supérieure et inférieure; avant utilisation par vaporisation sur le visage, on agite la lotion biphase.
Cette lotion biphase huileuse n'est pas grasse mais légère et évanescente. Elle pénètre vite et laisse la peau nette, douce et mate.

### Exemple 3 : Soin hydratant biphasé pour peaux mixtes

| *phase aqueuse* | |
|---|---|
| glycérol | 5% |
| conservateurs | qs |
| eau | qsp 100% |

| *phase huileuse* | |
|---|---|
| 4- Trifluoromethyl perfluoromorpholine | 35% |
| cyclopentasiloxane | 15% |

Ce produit biphasé apporte une sensation immédiate de fraîcheur et de bien-être. La peau reste souple, fraîche et mate.

### Exemple 4 : Fond de teint matifiant

| *phase A* | |
|---|---|
| dimethicone copolyol acétyl methyl taurate | 5,4% |
| glycérol | 3% |
| gomme de xanthane | 0,2% |
| pigments | 7% |
| agents dispersants | 0,2% |
| agents épaississants | 0,5% |
| conservateurs | qs |
| eau | qsp 100% |

| *phase B* | |
|---|---|
| Nonafluorométhoxybutane | 10% |
| cyclohexasiloxane | 10% |

On disperse à froid sous agitation la phase B dans la phase A et l'on obtient un fond de teint matifiant d'application aisée et agréable.

## Revendications

1. Utilisation d'au moins une huile fluorée volatile dans une composition cosmétique, pour estomper et/ou camoufler les imperfections et/ou les défauts du relief de la peau tels que les rides, les ridules, les pores et/ou les microreliefs; et/ou pour matifier la peau; et/ou pour unifier et/ou éclaircir le teint.

2. Utilisation d'au moins une huile fluorée volatile pour la préparation d'une composition dermatologique, destinée à estomper et/ou camoufler les imperfections et/ou les défauts du relief de la peau tels que les rides, les ridules, les pores et/ou les microreliefs; et/ou à matifier la peau et/ou à unifier et/ou éclaircir le teint.

3. Utilisation selon l'une des revendications précédentes, dans une composition destinée au traitement des peaux grasses.

4. Utilisation selon l'une des revendications précédentes, dans laquelle l'huile fluorée volatile est choisie parmi, seul ou en mélange,
i) les perfluorocycloalkyles répondant à la formule (I) suivante : 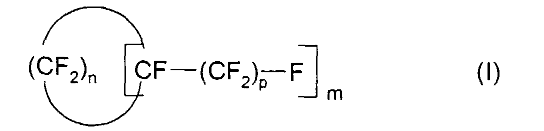 dans laquelle :
- n est 4 ou 5,
- m est 1 ou 2, et
- p est 1, 2 ou 3,
sous réserve que lorsque m = 2, les groupements ne sont pas nécessairement en alpha, l'un par rapport à l'autre;
ii) les perfluoroalcanes répondant à la formule (Il) suivante :
CF₃-(CF₂)ₘ-CF₂X (II)
dans laquelle m est 2 à 8 et X représente Br ou F;
iii) les fluoroalkyles ou hétérofluoroalkyles répondant à la formule (III) suivante :
CH₃-(CH₂)ₙ-[Z]ₜ-(CF₂)ₘ-CF₃ (III)
dans laquelle :
- t est 0 ou 1,
- n est 0, 1, 2 ou 3,
- m est 2, 3, 4 ou 5, et
- Z est O, S ou NR avec R représentant H, -(CH₂)ₘ-CH₃ ou -(CF₂)ₘ-CF₃.
iv) les dérivés de perfluoromorpholine répondant à la formule (IV) : 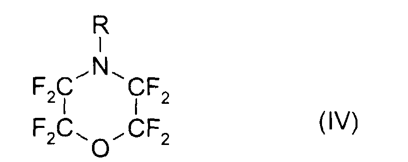 dans laquelle R est un radical perfluoroalkyle en C1-C4.

5. Utilisation selon l'une des revendications précédentes, dans laquelle l'huile fluorée volatile est choisie parmi, seul ou en mélange, le perfluorométhylcyclopentane, le perfluoro-1,3-diméthylcyclohexane, le dodécafluoropentane, le tétradécafluorohexane, le bromoperfluorooctyle, le nonafluorométhoxybutane, le nonafluoroéthoxyisobutane, la 4-trifluorométhyl perfluoromorpholine.

6. Utilisation selon l'une des revendications précédentes, dans laquelle l'huile fluorée volatile est présente dans la composition à raison de 0,5 à 100% en poids, notamment 3 à 40% en poids et de préférence 5 à 20% en poids.

7. Utilisation selon l'une des revendications précédentes, dans laquelle la composition se présente sous forme d'une émulsion huile-dans-eau, eau-dans-huile ou multiple, plus ou moins fluide; d'un produit anhydre tel que solution, dispersion ou gel anhydre lipophile, monophase, biphase ou multiphase.

8. Utilisation selon l'une des revendications précédentes, dans laquelle la composition se présente sous la forme d'un produit coloré ou non coloré, contenant éventuellement des actifs cosmétiques ou dermatologiques.

9. Utilisation selon l'une des revendications précédentes, dans laquelle la composition se présente sous forme d'un produit de maquillage de la peau du visage, du corps ou des lèvres, tel qu'un fond de teint, un fard à joues ou à paupières, un stick anti-cernes, un eye-liner, un mascara, un rouge à lèvres; sous forme d'un produit de soin dermatologique ou cosmétique de la peau du visage, du corps y compris le cuir chevelu, des lèvres, tel qu'une base de soin pour les lèvres (baumes à lèvres, protégeant les lèvres du froid et/ou du soleil et/ou du vent), une base fixante à appliquer sur un rouge à lèvres classique, notamment pour en limiter le transfert; une composition de protection solaire ou de bronzage artificiel; un produit déodorant; une composition de soin de jour pour le visage; une composition matifiante pour le visage.

10. Procédé de traitement cosmétique de la peau du visage, du corps y compris le cuir chevelu, des lèvres, destiné à lui conférer un aspect plus mat et/ou à estomper et/ou camoufler les imperfections et/ou les défauts du relief de la peau tels que les rides, les ridules, les pores et/ou les microreliefs; et/ou à unifier et/ou éclaircir le teint, caractérisé par le fait qu'on applique sur la peau une quantité efficace d'une composition cosmétique comprenant au moins une huile fluorée volatile.
